# EUROPEAN PATENT APPLICATION

(11) **EP 4 378 433 A1**
(43) Date of publication of application: **05.06.2024**
(21) Application number: 23211671.5
(22) Date of filing: 23.11.2023
(51) Int. Cl.: A61F 9/009

(54) **OPHTHALMOLOGICAL PATIENT INTERFACE AND METHOD OF MANUFACTURING A OPHTHALMOLOGICAL PATIENT INTERFACE**

(30) Priority: 28.11.2022 CH 14292022
(71) Applicant: Ziemer Ophthalmic Systems AG, 2562 Port (CH)
(72) Inventor: KAISER, Nikolaus, 2533 Evilard (CH); RATHJEN, Christian, 28197 Bremen (DE); STEINLECHNER, Michael, 8006 Zürich (CH)
(74) Representative: Rentsch Partner AG

(57) **Abstract**

The present disclosure relates to an ophthalmological patient interface (6) for application to an eye (91) of a patient (9) and to a method of manufacturing the ophthalmological patient interface (6), the ophthalmological patient interface (6) comprising a coupling portion (61), an eye fixation portion (62) and a passage (63), extending through the coupling portion (61) and the eye fixation portion (62), wherein the passage (63) is configured to enable a treatment laser beam (T) from a laser applicator (5) to pass through the ophthalmological patient interface (6), wherein at least one of: the coupling portion (61) or the eye fixation portion (62) comprises a patient-specific portion (65), shaped individually with respect to at least one of: a surface shape of the eye (91) of the patient (9) or a surface shape of a surrounding of the eye (91) of the patient (9).

## Description

### FIELD OF THE DISCLOSURE

The present disclosure relates to an ophthalmological patient interface for application to an eye of a patient and to a method of manufacturing the ophthalmological patient interface. In particular, the present disclosure relates to an ophthalmological patient interface and to a method of manufacturing the ophthalmological patient interface, wherein at least one of: the coupling portion or the eye fixation portion comprises a patient-specific portion.

### BACKGROUND OF THE DISCLOSURE

Ophthalmological treatment devices, which use a laser for eye treatment, are known. The ophthalmological treatment device has a laser source, which produces a pulsed laser beam. The wavelength of the laser light produced by the ophthalmological treatment device is dependent on the type of eye treatment and is typically in the ultraviolet (190nm to 230nm) or infrared (780nm to 1100nm) range.

The laser beam is typically produced by a laser source arranged in a base station. The laser beam is then guided along a beam path to a laser applicator, where the laser beam is focused onto a patient's eye. The laser applicator can be movably connected to the base device, for example by way of an articulated arm, wherein the articulated arm may simultaneously serve for optical beam guidance from the laser light source to the laser applicator.

Mechanical and optical coupling of the laser applicator to the patient eye, for example to the cornea of the patient eye, is conventionally carried out by way of a patient interface, wherein the patient interface may comprise a transparent contact body, through which the laser pulses emerging from the projection lens are guided and which, by way of a mechanical contact with the cornea of the eye, fixes the latter with respect to the patient interface and the laser applicator.

The human eye and in particular the cornea of the human eye does not have a perfect spherical shape. Each eye has a different shape, which deviates at least partially from the perfect spherical shape.

The available conventional patient interfaces are standard parts available in a single size. This patient interface comprises a rotationally symmetric contact surface, which is configured to contact the eye of the patient. The rotationally symmetric contact surface has a flat circular or ring circular shape and deforms the eye of the patient to achieve the desired contact over the whole available contact surface. The standard patient interface is therefore never perfectly aligned with a specific eye of a patient. This could lead to an unstable coupling of the patient interface to the eye, an undesired decoupling of the patient interface during eye treatment or to an injury of the eye due to a strong deformation of the eye of the patient. Further, the available standard patient interface could have a contact conflict with an eye-surrounding surface of the patient. For example, a big nose or face deformities could make it impossible to apply the standard patient interface on the eye of the patient for treatment.

### SUMMARY OF THE DISCLOSURE

It is an object of the present disclosure to provide an ophthalmological patient interface for application to an eye of a patient and to provide a method of manufacturing an ophthalmological patient interface. In particular, it is an object of the present disclosure to provide an ophthalmological patient interface and a method of manufacturing the ophthalmological patient interface, which does not have at least some of the disadvantages of the prior art.

According to the present disclosure, these objects are addressed by the features of the independent claims. In addition, further advantageous embodiments follow from the dependent claims and the description.

According to the present disclosure, an ophthalmological patient interface for application to an eye of a patient is specified. The ophthalmological patient interface comprises a coupling portion configured to be arranged on a laser applicator of an ophthalmological laser treatment system, an eye fixation portion configured to be arranged on the eye of the patient and a passage, extending through the coupling portion and the eye fixation portion. The passage is configured to enable a treatment laser beam from the laser applicator to pass through the ophthalmological patient interface. The coupling portion and / or the eye fixation portion comprises a patient-specific portion, shaped individually with respect to a surface shape of the eye of the patient and / or a surface shape of a surrounding of the eye of the patient. The patient-specific portion is for example a part or a section of the coupling portion and / or the eye fixation portion or the entire coupling portion and / or the entire eye fixation portion. Further, the patient-specific portion may form the entire ophthalmological patient interface. The patient is for example a mammal, in particular a human, a dog or a cat. Further, the surface shape of the eye of the patient, which may be the basis for the patient-specific portion of the ophthalmological patient interface, may be the surface shape of a donor eye. In other words, a surface shape of a donor eye, which is for example treated by the ophthalmological laser treatment device to provide eye tissue for a cornea transplantation, determines the shape of the patient-specific portion. Another designation of patient-specific portion is patient-matched portion.

The patient-specific portion of the ophthalmological patient interface enables to position the ophthalmological patient interface perfectly aligned on the individually formed eye of the patient and / or perfectly aligned with respect to the individually formed shape of the eye surrounding surface of the patient. The ophthalmological patient interface according to the present disclosure reduces or avoids undesired detachments during treatment and reduces or avoids injuries of the eye. Further, with the ophthalmological patient interface according to the present disclosure it is possible to treat eyes of patients, which are not treatable with a standard conventional ophthalmological patient interface.

In an embodiment, the patient-specific portion is individually shaped with respect to the shape of the nose of the patient, the shape of the eye sockets of the patient and / or the shape of deformities of the face of the patient, which surround the eye. The surface shape of the surrounding of the eye of the patient comprises the shape of the nose of the patient, the shape of the eye socket of the patient and / or the shape of deformities of the face of the patient. These eye surrounding, for example, a big nose of the patient, deep eye sockets of the patient or other face deformities, for example due to an accident, may limit the usage of a standard patient interface. Using the ophthalmological patient interface comprising the patient-specific portion, which is individually shaped based on the above-mentioned surroundings of the eye of the patient, enables to extend the treatment possibilities.

In an embodiment, the patient-specific portion is individually shaped with respect to a geometrical shape of the eye of the patient, in particular with respect to a diameter of the eye of the patient, a curvature radius at a planned contact position of the eye of the patient, an irregular curvature of the cornea of the eye of the patient, an irregular curvature of the limbus of the eye of the patient and / or an irregular curvature of the sclera of the eye of the patient. The geometrical dimensions of the eye of the patient define the surface shape of the eye. In particular, the above-mentioned geometrical dimensions and irregularities define the actual patient individual surface shape of the eye of the patient. Taking into account at least some of the above-mentioned geometrical dimensions and irregularities advantageously improves the individual shape of the patient-specific portion of the ophthalmological patient interface with respect to the individual shape of the eye of the patient. For example, the patient-specific portion has at least partially the complementary shape of the individual surface of the eye, onto which the ophthalmological patient interface is designed to be placed. Thereby, corneal astigmatisms, or other malalignments or disease of the eye may be advantageously compensated.

In an embodiment, the coupling portion and the eye fixation portion are two separate parts of the ophthalmological patient interface, which are configured to be coupled together to form the ophthalmological patient interface, wherein the eye fixation portion comprises the patient-specific portion. The coupling portion and the eye fixation portion are for example coupled together via an engaging mechanism, which forms, for example, a form fit coupling between these two parts. In an embodiment, the coupling portion is for example a standard part (off the shelf) and the individually shaped eye fixation portion, comprising the patient-specific portion, is configured to be coupled to the coupling portion to form the ophthalmological patient interface. In another embodiment, the coupling portion comprises the patient-specific portion, for example individually shaped with respect to the surface shape of the surrounding of the eye of the patient. In this embodiment, the eye fixation portion may be a standard part (off the shelf) and the coupling portion comprises the patient-specific portion. An individually shaped coupling portion and an individually shaped eye fixation portion is also conceivable.

In an embodiment, the patient-specific portion of the ophthalmological patient interface is at least partially or entirely made of bio-compatible and / or sterilizable material. In a further embodiment, the entire ophthalmological patient interface is made of a sterilizable material.

In an embodiment, the ophthalmological patient interface is at least partially made of metals, for example stainless steel or titan. In a further embodiment, the ophthalmological patient interface may comprise an oxidized metal surface.

In another embodiment, the ophthalmological patient interface is at least partially made of thermoplastics in particular of medical grade, for example: glycol-modified polyethylenterephthalat (PETG), polymethylmethacrylat (PMMA), low density polyethylene (LDPE), high density polyethylene (HDPE), polypropylene (PP), polyamide (PA), polyetheretherketon (PEEK, e.g. KetaSpire^{®}), polyetherimide (PEI), acrylnitril-butadien-styrol-copolymer (ABS), polycarbonate (PC, e.g. lexan^{®}, Makrolon^{®}), polyetheretherketon (PAEK, e.g. AvaSpire^{®}), polyacrylamide (PARA, e.g. Ixef^{®}), polyphenylsulfone (PPSU, e.g. Radel^{®}), polysulfone (PSU, e.g. Udel^{®}), polydimethyl siloxane (PDMS) or a combination thereof.

In another embodiment, the ophthalmological patient interface is at least partially made of thermoplastic elastomers, in particular of medical grade.

In another embodiment, the ophthalmological patient interface is at least partially made of a light-curing plastic (photopolymer), for example acrylic, epoxy or vinyl ester resin. These materials are for example used in a bath-based photopolymerization process for manufacturing of the patient-specific portion.

In another embodiment, the ophthalmological patient interface is at least partially made of ceramic materials, for example glass based systems (mainly silica), glass-based systems (mainly silica) with fillers, usually crystalline (typically leucite or, more recently, lithium desilicated), Crystalline-based systems with glass fillers (mainly alumina) or polycrystalline solids (alumina and zirconia).

In an embodiment, the materials as described above are manufactured or treated in a quality that they still act as medical safe devices, therefore each raw material can be suitable by itself, but can change during manufacturing or in connection with other materials or environments, such that a safe application is no longer guaranteed.

In an embodiment, the ophthalmological patient interface is made of materials and is made using known manufacturing processes, which in combination provide a high probability that the resulting material is suitability for the required application in the patient-specific portion of the ophthalmological patient interface. The manufacturing processes may comprise finishing steps, which may turn biological incompatible materials into compatible materials. For example, tetrahydrofuran is used as solvent for ophthalmological patient interface devices during a coating process and is in its liquid form hazardous to mucosal tissue (e.g. eye tissue). Due to hardening and evaporation, the remains are totally compatible and suitable for application on eye tissue. In another embodiment, a polymer, which is in its raw form completely inert, can turn hazardous due to usual manufacturing processes with ray or gas treatment. Since polymer-deformed molecule cracks can occur and release unwanted emissions such as formaldehyde for example. Therefore, the testing and validation of the finished product is of high importance.

In an embodiment, the eye fixation portion comprises an at least partially transparent contact body, which is arranged in the passage at the eye facing end of the eye fixation portion, and which is configured to contact the cornea and / or the sclera of the eye, wherein the contact body comprises the patient-specific portion. In other words, the contact body, which forms part of the eye fixation portion, comprises the patient-specific portion. The contact body has for example an applanating or a non-applanating shape. The applanating contact body is configured to deform the surface of the eye of the patient and the non-applanating contact body is configured to conform / align with the surface of the eye of the patient. Deforming the surface of the eye, for example the surface of the cornea of the eye, is critical, deforming a surface of the eye which has deformities, for example a strong astigmatism is even more critical. The patient-specific portion of the contact body can take into account the individual shape of the surface of the eye, for example the individual irregularities of the cornea of the eye of the patient. This results in an advantageous application of the contact body on the surface of the eye, in particular using the patient-specific applanating contact body. In an embodiment, the contact body comprising the patient-specific portion is a separate part arranged at the eye fixation portion. In another embodiment, the contact body comprising the patient-specific portion is integrally formed with the eye fixation portion.

In an embodiment, the contact body is made of glass or the same material as the eye fixation portion. In an embodiment, the contact body is made of at least one of the materials described above with respect to the patient-specific portion of the ophthalmological patient interface, in particular of an at least partially transparent material.

In an embodiment, a geometrical extension of the patient-specific portion is limited to a predefined enveloping geometry at least partially surrounding the ophthalmological patient interface. The predefined enveloping geometry defines / limits for example the maximum possible extension of an eye contact surface of the eye fixation portion towards a specific direction. The enveloping geometry is for example a virtual box or sphere surrounding the ophthalmological patient interface in which the patient-specific portion may extend until it reaches the inner border of the virtual box or sphere. Other shapes of the enveloping geometry are also conceivable. For example, the enveloping geometry may also be a plane surface. The enveloping geometry does not need to completely envelop the ophthalmological patient interface; partially enveloping or partially limiting is also conceivable. In an embodiment, the enveloping geometry comprises specific predefined parameter ranges for different features of the ophthalmological patient interface. The different features are; for example, suction openings, which need a minimal effective suction surface for advantageous positioning, another example is the diameter of the passage, which should, for example, not fall below a predefined minimal value. Analogously, there is a maximal diameter of the human eye. Additional parameters or parameter ranges for other features of the ophthalmological patient interface are also conceivable, for example curvature radii. Further, the enveloping geometry may be at least partially determined using an eye model, for example the Gullstrand model eye.

In an embodiment, the enveloping geometry defines or comprises representative intermediate variables or ranges, which cover predefined parameter ranges for different dimensions of the patient-specific portion. Further, individual customizations of the patient specific portion are for example, interpolations of the predefined parameter ranges or a combination of interpolations.

The approval process of different medical parts requires sometimes many different steps, in particular, in case the medical device is configured to contact the human body. It could be that each individually shaped part needs to go through the approval process. The patient-specific portion of the ophthalmological patient interface could require to go through the approval process for each individually shaped ophthalmological patient interface. The enveloping geometry enables to meet automatically the approval requirements for each patient-specific portion of the ophthalmological patient as long as it is shaped within the enveloping geometry. For example, the approval process for the ophthalmological patient interface is performed for the enveloping geometry, such that each patient-specific portion, which is arranged within the enveloping geometry, meets automatically the approval / certification requirements.

In an embodiment, the enveloping geometry has the shape of a standard ophthalmological patient interface plus a predefined extension in at least one direction, preferably in all directions, wherein the extension is in the range from 0,05 mm to 20 mm, preferably in the range from 0.1 mm to 5 mm.

In an embodiment, the enveloping geometry is determined prior to the determination of the patient specific portion. The enveloping geometry may comprise minimum and maximum dimensions, mechanical performance limits and / or other clinically relevant factors for the required application on the eye of the patient.

In an embodiment, the patient specific portion is accomplished or designed by specific techniques, in particular software based techniques, such as scaling of at least one dimension or group of dimensions of a base model of the ophthalmological patient interface. The dimensions to be scaled are, for example, selected based on the surface shape of the eye and/or a surface shape of the surrounding of the eye of the patient. In a further embodiment, the patient-specific ophthalmological patient interface is designed using full anatomic features of the eye and/or the surrounding received from an imaging device.

In an embodiment, the dimensions to be varied of the patient-specific portions are categorized, for example, in a first and second category, the first category comprises clinically relevant dimensions, for example, dimensions, which define the eye contacting surface, the second category comprise clinically not relevant dimensions, for example, dimensions, which define a distance to surroundings of the eye. The dimensions of the first category may have different tolerance and approval requirements than the dimensions of the second category. The patient specific portion is advantageously designed using a manipulation software, which already comprises the predefined enveloping geometry and / or further limitations. Any software or procedure used to make modifications to the patient specific design based on clinical input (shape of the eye and / or shape of the surrounding) should include internal checks that prevent the operator from exceeding the pre-established device specifications, which are for example documented in a device master record.

In an embodiment, the eye fixation portion comprises a rotationally asymmetric contact surface, which is formed by the patient-specific portion, wherein the rotationally asymmetric contact surface is configured to contact the sclera and / or the cornea of the eye of the patient. Rotational symmetry, also known as radial symmetry, in geometry, is the property a shape has when it looks the same after some rotation by a partial turn. Rotational asymmetry on the contrary is, according to this embodiment, the property a shape has when it looks not the same after some rotation by a partial turn. Certain geometric objects are partially symmetrical, but still rotational asymmetric, when rotated at certain angles such as squares rotated 90°, however the only geometric objects that are truly rotationally symmetric at any angle are spheres, circles, spheroids and solids of revolution. The rotationally asymmetric contact surface provides the advantageous possibility to apply the ophthalmological patient interface non-coaxially with the optical axis of the eye. The ophthalmological patient interface can be applied, for example, laterally next to the optical axis of the eye, which substantially increases the possible treatment surface for the treatment laser beam.

The optical axis of the eye is for example the straight line between the centers of curvature of refractive surfaces of the eye. The optical axis of the eye may further be the visual axis of the eye, which runs from the fovea centralis of the eye through the nodal point of the eye to the object of fixation. The optical axis of the eye may further be the line of sight of the eye, which is the straight line from the fixation point reaching the fovea centralis through the pupil center. The optical axis of the eye may further be the pupil axis, which is the straight line between the center of the cornea and the center of the pupil. Angular deviations of this axis are usually less than 5 degrees. The optical axis may further be a geometrical combination (best fit) between two or more of the above-mentioned different axis of the eye.

The asymmetric contact surface for a decentralized application on the eye of the patient is in particular simple an advantageous realizable by the patient-specific portion. For example, the patient of the eye may comprise a disease, like a pterygium, which is located laterally next to the cornea of the eye at a specific position. An advantageous alignment of the patient interface above the center of the pterygium, for an advantageous treatment of the pterygium, is realizable by the patient-specific portion shaped with respect to the orientation and location of the pterygium itself. In other words, the patient-specific portion of the ophthalmological patient interface is shaped based on the position and orientation of the pterygium to be treated. The asymmetric contact surface for the desired decentralized application, for example centrally above the pterygium, is in particular easily and advantageously realizable by the patient-specific portion.

In a further embodiment, the patient-specific portion is a single part, which forms part of the coupling portion or the eye fixation portion, and which is configured to be attached / mounted to the eye fixation portion or the coupling portion.

In an embodiment, the eye fixation portion of the ophthalmological patient interface comprises a suction opening configured to be fluidically connected to a negative pressure, wherein the patient-specific portion forms the shape of the suction opening. In other words, the suction opening is at least partially the patient-specific portion. For example, different surface structures of the eye of the individual patient may require to individually position the suction openings of the ophthalmological patient interface at an individual position, for an advantageous application of the ophthalmological patient interface on the eye of the patient. In a further embodiment, the ophthalmological patient interface comprises at least one additional opening extending through the eye fixation portion and/or coupling portion. The at least one additional opening is, for example, configured to enable access for instruments, or to inject or extract fluids, in particular into the passage.

In an embodiment, the eye fixation portion comprises protrusions, which are configured to penetrate tissue of the sclera or the cornea respectively of the eye of the patient, and wherein the patient-specific portion defines the shape and / or the position of the protrusion on the eye fixation portion. In an embodiment, the protrusions are spike shaped and extend from the eye fixation portion. The protrusions have for example a pyramid shape. In a further embodiment, the protrusions are arranged only on the area of the eye fixation portion, which is configured to be placed on the sclera of the eye of the patient. The sclera tissue is relatively soft compared to the cornea tissue, which is advantageous for the required penetration of the spikes in the sclera tissue to position the ophthalmological patient interface rigidly on the eye.

In an embodiment, the ophthalmological patient interface comprising the patient-specific portion, further comprises a patient identifier, which is configured to identify the ophthalmological patient interface to the patient. In other words, the patient identifier connects the individually shaped ophthalmological patient interface to the patient. The patient identifier is for example a code (QR-code, bar-code) directly integrated in the ophthalmological patient interface or attached to the ophthalmological patient interface. The code is, for example, manufactured on the ophthalmological patient interface during, prior or after the manufacturing of the patient-specific portion. In another embodiment, the patient identifier is a radio frequency identification tag, attached on the ophthalmological patient interface or on a packaging of the ophthalmological patient interface. For example, it may be required to scan or identify the ophthalmological patient interface, which is determined to be arranged on the laser applicator, prior to the eye treatment of the patient, in order to ensure that the right patient specific ophthalmological patient interface is used for the right patient. It is important to ensure that the right ophthalmological patient interface is used for the right patient. This may be in particular important because days or weeks can be in between manufacturing and using of the ophthalmological patient interface.

In a further aspect of the present disclosure, a method of manufacturing an individually shaped ophthalmological patient interface is specified. The method comprising the following steps:
- measuring, by a measuring device, the surface of the eye of the patient and / or the eye surrounding surface of the patient;
- determining, by a computing device, the shape of the surface of the eye of the patient and / or the shape of the eye surrounding surface of the patient;
- manufacturing, by a manufacturing device, a patient-specific portion of the coupling portion and / or the eye fixation portion, based on the determined shape of the surface of the eye and / or the determined shape of the eye surrounding surface.

The measuring device is for example an optical coherence tomography device or a triangulation device, which measures in a preparatory step the surface of the eye and / or the eye surrounding surface of the patient. The measurement data is afterwards for example sent to the computing device. The measurement device and the computing device are for example one single device. In other words, the measurement device may also act as computing device and determines the shape of the surface of the eye and / or the shape of the eye surrounding surface using the measurement data. The computing device is for example a specific processor for the determination of the shape of the surface of the eye and / or the shape of the eye surrounding surface of the patient. The shape of the eye surrounding surface is for example the shape of the nose of the patient, the shape of the eye socket of the patient and / or the shape of deformities of the face of the patient.

In an embodiment, the step of manufacturing comprises adding material, by an additive manufacturing device, for forming the patient-specific portion. The additive manufacturing device is for example a 3D printer or any other device, which is configured to manufacture the patient-specific portion by addition material. The coupling portion and / or the eye fixation portion comprising the patient-specific portion is / are for example completely additively manufactured. The additive manufacturing device is for example configured to use a binder jetting technology, a direct energy deposition technology, a material extrusion technology, a powder-bed fusion technology, a sheet lamination technology, a vat polymerization technology and / or a direct energy deposition-arc technology.

In an embodiment, the method further comprises the steps of providing a blank coupling portion and / or a blank eye fixation portion and additively manufacturing, by the additive manufacturing device, the patient-specific portion onto the blank coupling portion and / or the blank eye fixation portion, by adding material onto the blank portion(s). The blank coupling portion, the blank eye fixation portion or a blank ophthalmological patient interface is for example a standard part having a predefined shape, which is configured to be placed in the additive manufacturing device, and which is further configured such that the patient-specific portion is additively applicable by adding material on the provided blank part(s). Another word for the blank unmachined parts is blank or slug.

In an embodiment, manufacturing comprises removing material, by a removing device, for forming the patient-specific portion of the ophthalmological patient interface. The removing device is for example a grinding device, a milling device or another material cutting manufacturing device. In an embodiment, the material removing, by the removing device, is performed after the additive manufacturing, for example, as a finishing step of the patient-specific portion.

In an embodiment, the method further comprises the step of manufacturing, by the removing device, the patient-specific portion out of the blank coupling portion and / or the blank eye fixation portion, by removing material from the blank unmachined portion(s). An unmachined eye fixation portion or an unmachiend coupling portion is a part, which has not been processed by the material removing manufacturing step. In an embodiment, the unmachined coupling portion and / or the unmachined eye fixation portion has at least partially the extensions and / or the shape of the enveloping geometry.

In an embodiment, the method further comprises the step of post processing of the manufactured patient specific portion of the ophthalmological patient interface. Post processing may comprise heat treatment, removing of manufacturing residues and / or final machining.

In an embodiment, the step of measuring is performed by an optical coherence tomography device, a Scheimpflug tomography device, a placido topography device, an optical biometer device, a 3D laser scanner device, a time of flight sensor, a structured illumination device and / or a combination thereof. The measuring step provides the required data of the face of the patient and / or of the eye of the patient for determining the shape of the patient-specific portion.

In an embodiment, the method further comprises the step of validating, by a certification software and / or a quality validation device at least the quality of the manufactured patient-specific portion of the individually shaped ophthalmological patient interface. For example, the certification software is configured to monitor the measuring device during the measuring step, the computing device during the determining step and / or the manufacturing device during the manufacturing step. Alternatively or additionally, the quality validation device validates the quality of the manufactured patient-specific portion, for example, by measuring the resulting patient-specific portion and comparing it to a virtual model of the patient-specific portion, for example via a variance analysis. In another embodiment, the quality validation device comprises a mold, a cast or a negative of the patient-specific portion. In this embodiment, the validation step may comprise to compare the manufactured patient-specific portion with the mold etc. for example by holding the manufactured patient-specific portion onto the mold.

In an embodiment, the step of validating, by the quality validation device, comprises mechanical testing, optical testing and / or dimensional testing of the manufactured patient-specific portion of the ophthalmological patient interface. In this embodiment, the patient specific portion is validated/approved after being manufactured. This is, compared to the surveillance of the entire manufacturing process, cheaper and less complex.

### BRIEF DESCRIPTION OF THE DRAWINGS

The herein described disclosure will be more fully understood from the detailed description given herein below and the accompanying drawings, which should not be considered limiting to the invention described in the appended claims. The drawings in which:
- **Figure 1**: shows a perspective view illustrating schematically an ophthalmological laser treatment system;
- **Figure 2**: shows a block diagram illustrating schematically an ophthalmological laser treatment system with an ophthalmological patient interface;
- **Figure 3**: shows schematically different components of the ophthalmological laser treatment system including the ophthalmological patient interface;
- **Figure 4**: shows schematically a cross section of a first conventional ophthalmological patient interface applied on an eye;
- **Figure 5**: shows schematically a cross section of a second conventional ophthalmological patient interface applied on an eye;
- **Figure 6**: shows schematically a perspective view of an ophthalmological patient interface according to a first embodiment;
- **Figure 7**: shows schematically a perspective view of the ophthalmological patient interface according to the first embodiment applied on an eye of a patient;
- **Figure 8**: shows schematically a perspective view of the ophthalmological patient interface according to the first embodiment applied on the eye and engaged with a laser applicator;
- **Figure 9**: shows schematically a top view of an ophthalmological patient interface according to a second embodiment applied on the eye;
- **Figure 10**: shows schematically a cross section of an ophthalmological patient interface according to a third embodiment;
- **Figure 11**: shows schematically a cross section of an ophthalmological patient interface according to a fourth embodiment;
- **Figure 12**: shows schematically a cross section of an ophthalmological patient interface according to a fifth embodiment;
- **Figure 13**: shows schematically a cross section of an ophthalmological patient interface according to a sixth embodiment;
- **Figure 14**: shows schematically a perspective view of an unmachined ophthalmological patient interface according to a seventh embodiment;
- **Figure 15**: shows schematically a perspective view of the ophthalmological patient interface of Figure 14 during manufacturing of a patient-specific portion;
- **Figure 16**: shows schematically a perspective view of an unmachined ophthalmological patient interface according to an eighth embodiment;
- **Figure 17**: shows schematically a perspective view of the ophthalmological patient interface of Figure 16 during manufacturing of a patient-specific portion;
- **Figure 18**: shows a block diagram of a method of manufacturing an ophthalmological patient interface according to a first embodiment;
- **Figure 19**: shows a block diagram of a method of manufacturing an ophthalmological patient interface according to a second embodiment.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

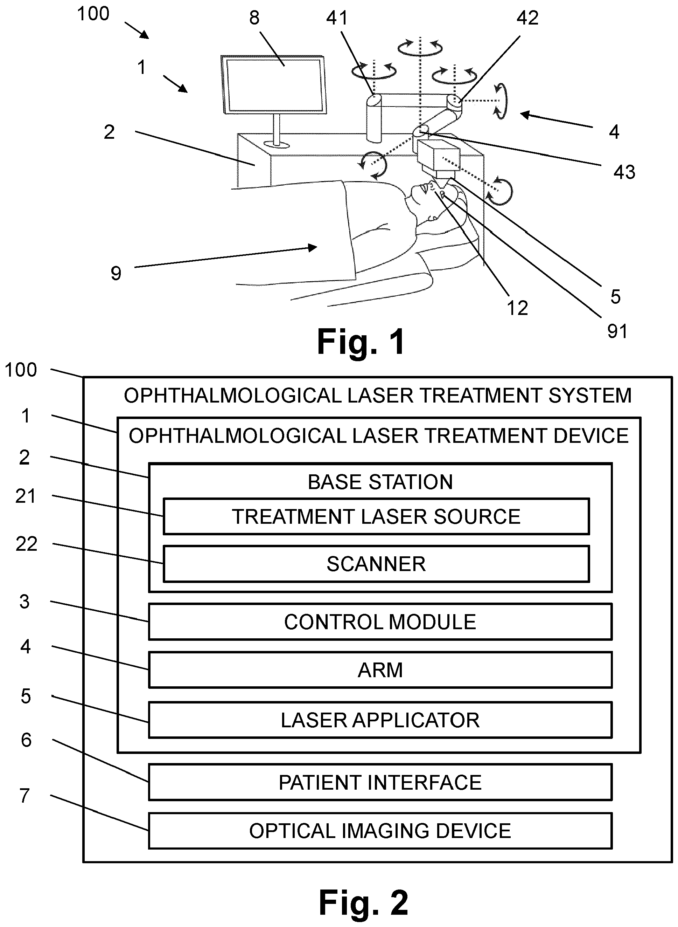

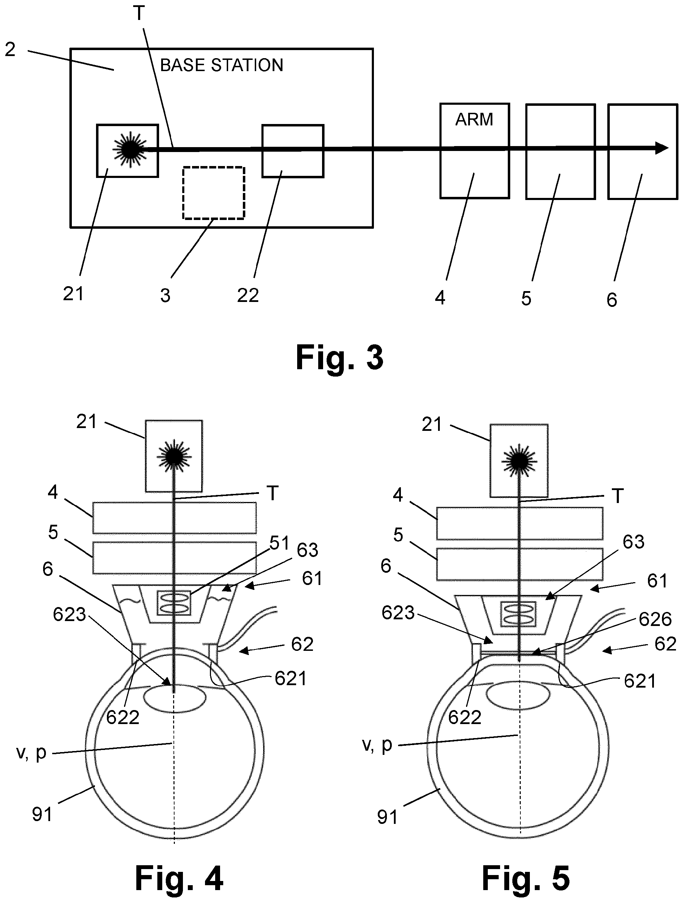

**Figure 1** shows a perspective view illustrating schematically an ophthalmological laser treatment system 100. **Figure 2** shows a block diagram illustrating schematically the ophthalmological laser treatment system 100 with an ophthalmological patient interface 6. **Figure 3** shows schematically different components of the ophthalmological laser treatment system 100 including the ophthalmological patient interface 6.

The Figures 1 to 3, schematically illustrate modules and/or elements of various embodiments of the ophthalmological laser treatment system 100 and provide exemplary sequences or arrangement of modules and/or elements, including modules and/or elements in a beam path T. Some modules and/or elements shown in a particular Figure may be combined with modules and/or elements shown in another Figure.

The ophthalmological laser treatment system 100 comprises an ophthalmological laser treatment device 1 comprising a base station 2. The base station 2 is configured as a fixed or mobile apparatus. The ophthalmological laser treatment device 1 has a treatment laser source 21 arranged in the base station 2, which generates a treatment laser beam T. The base station 2 further includes, for example, a power supply and other auxiliary subsystems necessary for operation of the ophthalmological laser treatment device 1.

The treatment laser source 21 is configured, for example, to generate an ultraviolet or infrared treatment laser beam T having a wavelength of between 190 nm and 230 nm for ultraviolet laser beam and 780nm to 1100nm for infrared laser beam. For example, the treatment laser source 21 comprises an excimer or a solid-state laser, which produces such an ultraviolet treatment laser beam T. The excimer laser uses a combination of a noble gas and a reactive gas under high pressure and electrical stimulation to generate the treatment laser beam T. In particular, an excimer laser using argon as the noble gas and fluoride as the reaction gas may be used as the treatment laser source 21.

In an embodiment, the treatment laser beam T is a pulsed laser beam. In an embodiment, the treatment laser source 21 is configured to generate femtosecond laser pulses, which have pulse widths of typically from 10 fs to 1000 fs (1 fs = 10^-15 s).

The base station 2 includes a scanner 22, which is configured to steer the treatment laser beam T delivered by the treatment laser source 21 onto or into treatment points on a treatment pattern (comprising a laser trajectory).

The ophthalmological laser treatment device 1 comprises a laser applicator 5 or application head 5. The laser applicator 5 is designed to guide the treatment laser beam T into or onto an eye 91 of a patient 9 (as shown, for example, in Figure 1). The laser applicator 5, for this purpose, can comprise focusing optics 51 configured to focus the treatment laser beam T onto one or more treatment points inside or on the eye 91, in particular the cornea or the sclera for a pointwise tissue disruption or ablation. The focusing optics 51 comprise a lens system having one or more optical lenses. Depending on the embodiment, the focusing optics 51 comprise one or more movable or deformable lenses and/or a drive for moving the entire focusing optics in order to set and adjust the focal depth, or the treatment height, in the projection direction along the projection axis. Figure 1 further shows schematically an eye surrounding surface 12 of the patient 9. The eye surrounding surface 12 is, for example, the nose, the eye socket, the forehead and / or other parts of the face, which surround the eye and which might get in conflict with any part of the ophthalmological laser treatment system 100.

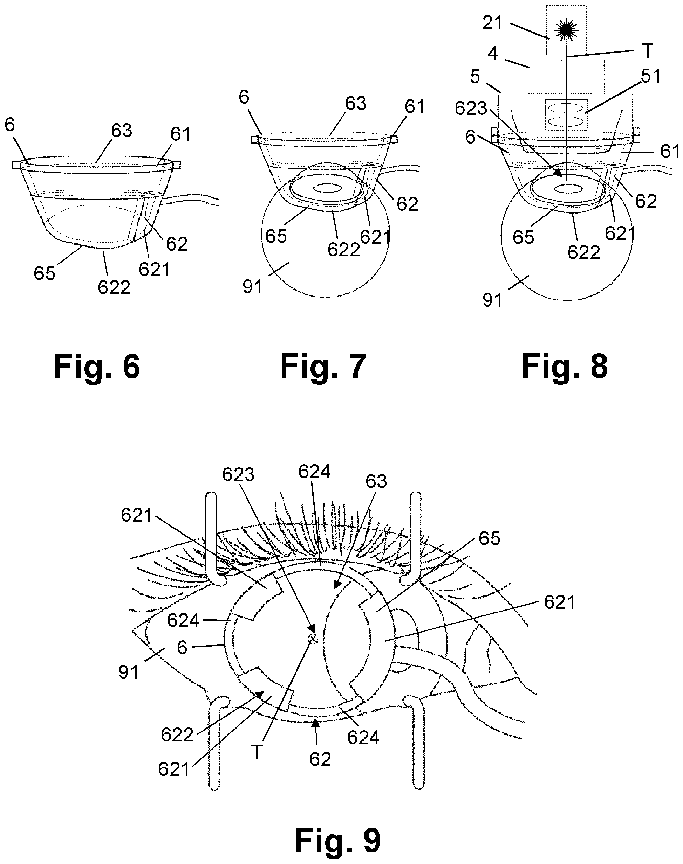

The ophthalmological laser treatment system 100 comprises an ophthalmological patient interface 6. The laser applicator 5 is preferably fixed onto the eye 91 by means of the ophthalmological patient interface 6, which is coupled to the eye for example using negative pressure. Different embodiments of the ophthalmological patient interface 6 of the present disclosure will be described in more detail with reference to the Figures 6 to 17.

Depending on the embodiment, the ophthalmological laser treatment system 100 further comprises an optical imaging device 7. The ophthalmological laser treatment device 1 comprises an arm 4 arranged between the base station 2 and the laser applicator 5. The arm 4 is configured to provide a beam path for the treatment laser beam T, such that the treatment laser beam T is guided along the inside of the arm 4 from the base station 2 to the laser applicator 5. In an embodiment, the arm 4 comprises one or more joints 41 (as shown in Figures 1) such that the laser applicator 5 is movable and/or rotatable with respect to the base station 2. Each rotatable joint 41 comprises a mirror arranged in the beam path to reflect the treatment laser beam T along the arm 4. The ophthalmological laser treatment system 100 is controlled by a control module 3, by controlling the treatment laser source 21 and the scanner 22, as well as by controlling additional modules of the ophthalmological laser treatment system 100 arranged in the beam path of the treatment laser beam T.

The ophthalmological laser treatment system 100 optionally includes a user interface comprising, for example, one or more user input devices, such as a keyboard, and one or more output devices, such as a display 8 (as shown in Figures 1). The display 8 may also be an input device. The user interface is configured to receive user inputs from an eye treatment professional, in particular based on, or in response to, information displayed to the eye treatment professional using the one or more output devices.

The **Figures 4** and **5** show schematically a cross section of a first and second conventional ophthalmological patient interface 6 applied on an eye 91 of a patient 9. The Figures 4 and 5 show schematically the treatment laser source 21, the arm 4 and the laser applicator 5 with the focusing optics 51. The Figures further show schematically the treatment laser beam T and its beam path from the treatment laser source 21 to the eye 91. The conventional ophthalmological patient interface 6 comprises a coupling portion 61, which is configured to be attached to the laser applicator 5, and an eye fixation portion 62, which is configured to contact the cornea of the eye 91. The eye fixation portion 62 comprises a contact surface 622, which is configured to contact the eye 91, when the ophthalmological patient interface 6 is applied on the eye 91. The Figures 4 and 5 further show schematically a ring shaped suction opening 621, which extends ring shaped along the entire contact surface of the eye fixation portion 62. The ring shaped suction opening 621 is connected to a negative pressure or vacuum such that the eye fixation portion 62 is rigidly attachable to the eye 91. The Figures 4 and 5 further show that the ophthalmological patient interface 6 comprises a passage 63, extending through the coupling portion 61 and the eye fixation portion 62. The treatment laser beam T is guided along the passage 63 to a treatment surface 623 on or in the cornea of the eye 91. The passage 63 is configured to enable the treatment laser beam T from the laser applicator 5 to pass through the ophthalmological patient interface 6 to penetrate a target volume of tissue of the eye 91. Figure 5 additionally comprises a contact body 626, which is arranged in the passage 63 at the eye facing end of the eye fixation portion 62, wherein the contact body 626 is configured to conform / deform the cornea of the eye 91, for example for a conventional laser vision correction LASIK surgery.

The Figures 4 and 5 further show the optical axis v of the eye 91 and an optical axis p of the ophthalmological patient interface 6. The passage 63 extends along the axis p. Both axis v, p are arranged coaxially with respect to each other. It is of high importance that these two axis are arranged coaxially with respect to each other for an optimal treatment result using the conventional ophthalmological patient interface 6 for the desired precise treatment. The coaxial alignment is achieved by a rotational symmetry of the contact surface 622 of the eye fixation portion 62, which aligns with the rotational symmetric surface of the cornea of an ideal eye 91.

Reference is now made to the embodiments of the present disclosure as shown in the Figures 6 to 17.

The **Figures 6, 7** and **8** show schematically three different views of an ophthalmological patient interface 6 according to a first embodiment of the present disclosure. Figure 6 shows the ophthalmological patient interface 6 comprising the coupling portion 61 and the eye fixation portion 62. The coupling portion 61 and the eye fixation portion 62 are according to this embodiment two separate parts, which is indicated in the figures. A passage 63 extends through the coupling portion 61 and the eye fixation portion 62. The eye fixation portion 62 further comprises a suction opening 621, which is configured to be fluidically connected to a negative pressure, such that the ophthalmological patient interface 6 is firmly positioned on the eye 91, when the ophthalmological patient interface 6 is applied on the eye 91 and the negative pressure is provided on the suction opening 621. The eye fixation portion 62 further comprises a patient-specific portion 65 shaped individually with respect to a surface shape of the eye 91 of the patient 9 and / or a surface shape of a surrounding of the eye 91 of the patient 9. The Figures 6 to 8 show that the surface of the eye fixation portion 62, which is configured to be in contact with the eye 91 of the patient 6 is not rotationally symmetric, but is individually shaped based on the surface shape of the eye 91. This individual shape is formed by a patient-specific shape 65 of the eye fixation portion 62. In other words, a contact surface 622 of the eye fixation portion 62 is complementary to the individual shape of the eye 91 of the patient 9. Figure 7 shows that the ophthalmological patient interface 6 comprising the patient-specific portion 65 is advantageously applicable on the eye 91 of the patient 9. Figure 8 additionally shows the ophthalmological patient interface 6 engaged with the application head 5, which comprises the focusing optics 51. Figure 8 further shows schematically the treatment laser source 21, the arm 4 and the treatment laser beam T, which is guided from the treatment laser source 21 to a treatment surface 623 of the eye 91.

Every eye 91 has an individual shape, which is determined by its surface, its different tissues, dimensions and individual diseases or disorders. The patient-specific portion 65, which is individually shaped with respect to the individual surface shape of the eye 91, provides an advantageous positioning of the ophthalmological patient interface 6 on the eye 91 due to its individually shaped patient-specific portion 65.

**Figure 9** shows schematically a top view of an ophthalmological patient interface 6 according to a second embodiment of the present disclosure applied on the eye 91. The ophthalmological patient interface 6 has on oval or egg shaped and is configured to be applied decentral on the eye 91 of the patient 9 with respect to the optical axis v of the eye 91 of the patient 9. Figure 9 does not show the entire ophthalmological patient interface 6, but only shows the contact surface 622 of the ophthalmological patient interface 6, determined by the patient-specific portion 65. Figure 9 advantageously show the rotational asymmetry of the contact surface 622 defined by the patient-specific portion 65 of the eye fixation portion 62, for an advantageous decentral application of the ophthalmological patient interface 6 on the eye 91. Figure 9 further show that the contact surface 622 comprises three suction openings 621, which further determines the rotational asymmetry of the contact surface 622. The shape and the position of the suction openings 621 may also be determined by the patient-specific portion 65 of the eye fixation portion 62. Figure 9 further shows seals 624 arranged between the suction openings 621. The patient-specific portion 65 determines the contact surface 622 comprising the seals 624 and the suction openings 621.

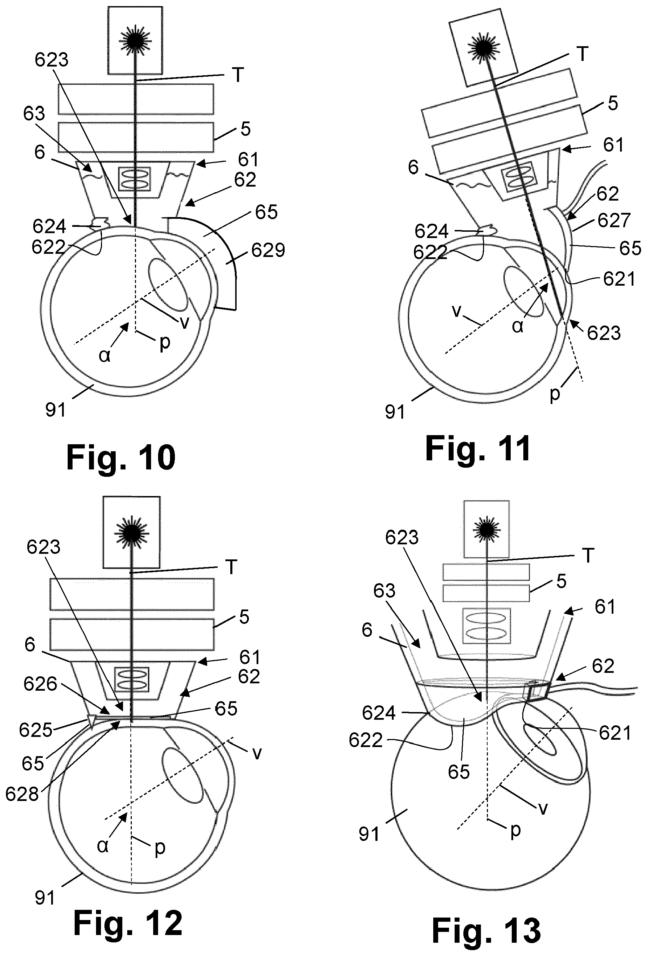

**Figure 10** shows schematically a third embodiment of the ophthalmological patient interface 6 according to the present disclosure. The ophthalmological patient interface 6 comprises a form fitted contact body 629. The patient-specific portion 65 of this embodiment comprises the form fitted contact body 629 arranged laterally next to the beam path of the treatment laser T. The form fitted contact body 629 extends laterally of the ophthalmological patient interface 6 next to the passage 63 and has a shape, which corresponds to a negative of the individual cornea of the eye 91 of the patient 9. When applied on the eye 91, the form fitted contact body 629 docks advantageously on the individual cornea of the eye 91, such that the ophthalmological patient interface 6 is advantageously rigidly attachable on the eye 91 of the patient. The surface tension of a tear film may provide an advantageous docking of the form-fitted contact body 629 on the eye 91, which further advantageously improves the docking of the entire ophthalmological patient interface 6 on the eye 91. The form-fitted contact body 629 determined by the patient-specific portion 65 may correspond to an individual aspheric shape of the cornea of the eye 91, resulting from an individual astigmatism.

**Figure 11** shows a cross section of an ophthalmological patient interface 6 according to a fourth embodiment of the present disclosure. The patient-specific portion 65 of the eye fixation portion 62 of the ophthalmological patient interface 6 enables to form a larger inclination angle α between the axis p and the optical axis v of the eye 91, when the ophthalmological patient interface 6 is applied on the eye 91. The patient-specific portion 65 of this embodiment comprises a sealing 624, one suction opening 621 and an extension portion 627, which extends from the coupling portion 61 beyond the maximal extension of the sealing 624. The extension portion 627, according to this embodiment, follows a radius such that the tip of the extension portion 627, which may comprise the suction opening 621, is advantageously applicable on the individual cornea of the eye 91. The patient-specific portion 65 may determine the length of the extension portions 627. The length of the extension portion 627 determines the inclination angle α between the axis p of the ophthalmological patient interface 6 and the axis v of the eye 91, when applied on the eye 91. The length of the extension portion 627 therefore also determines the angle of incidence of the treatment laser beam T. In Figure 11, the treatment surface 623, which is advantageous reachable by the treatment laser beam T, using this specific ophthalmological patient interface 6 comprising the extension portion 627, is the interior side of the ocular limbus of the eye 91. The treatment surface 623 of another embodiment (not shown), which is advantageous reachable by the treatment laser beam T, using another specific ophthalmological patient interface 6, is the anterior chamber angle of the eye 91.

**Figure 12** shows schematically a cross section of an ophthalmological patient interface 6 according to a fifth embodiment of the present disclosure. The ophthalmological patient interface 6 comprises an at least partially transparent contact body 626, arranged in in the passage 63 at the eye facing end of the eye fixation portion 62. The contact body 626 or contact glass is at least partially transparent for the treatment laser beam T to enable the penetration of the target volume of tissue of the eye 91. The contact body 626 comprises a contact surface 628, which forms part of the rotationally asymmetric contact surface 622 and which is configured to conform the sclera of the eye 91. These embodiments do not comprise a coupling liquid arranged in the passage 63 and also do not comprise a corresponding sealing, but may comprise a suction opening 621, preferably partially ring shaped, arranged laterally next to, and preferably at least partially enclosing, the contact body 626. Other embodiments, for example as shown in Figure 10 or 11, may comprise a coupling liquid. The contact body 626 is, according to this embodiment, configured to contact with its contact surface 628 the cornea and the sclera of the eye 91. The contact body 626 comprises according to this embodiment the patient-specific portion 65, which is for example determined by the individual surface shape of the sclera of the eye 91. The embodiment of the ophthalmological patient interface 6 shown in Figure 12 further comprises at least one protrusion 625, which forms part of the patient-specific portion 65 and which is configured to penetrate tissue of the sclera of the eye 91, when the ophthalmological patient interface 6 is applied on the eye 91. The protrusions 625 are for example spike shaped and are arranged laterally next to the contact body 626. The protrusions 625 have for example the shape of a pyramid. The protrusions 625 advantageously help to position the ophthalmological patient interface 6 rigidly on the eye 91. Sclera tissue is relatively soft and is not easily damaged by the protrusions 625, compared to cornea tissue. Nevertheless, in a further embodiment, at least one protrusion 625 could also be also configured to penetrate the cornea of the eye 91. The protrusions 625 are for example distributed individually unevenly around the contact surface 622, thereby taking into account the individual surface shape of the. In another embodiment, the protrusions 625 are evenly distributed around the contact surface 622.

In a further embodiment, not shown in the Figures, the eye fixation portion 62 may comprise protrusions 625 and at least one, preferably a plurality of suction openings 621.

**Figure 13** shows schematically a perspective view of an ophthalmological patient interface 6 according to a sixth embodiment of the present disclosure. Figure 13 shows in a perspective view the ophthalmological patient interface 6 applied on the eye 91. Figure 13 advantageously shows the individually shaped patient-specific portion 65, which determines a rotational asymmetry of the contact surface 622 of the eye fixation portion 62. The contact surface 622 as shown in this Figure is not only rotational asymmetric in a plane area but also varies in its vertical extension such that the contact surface 622 advantageously fits / advantageously touches the individual surface of the eye 91. The patient-specific portion 65 is according to this embodiment determined based on the individual shape of the surface of the eye 91 of the patient 9 and based on the planned application position of the ophthalmological patient interface 6 on the eye 91. For example, if the treatment surface 623 would be located on a different position, the patient-specific portion 65 would look different to enable the desired advantageous positioning. As visible in Figure 13, the extension length of the patient-specific portion 65 varies around the eye fixation portion 62, such that the contact surface 622 is applicable on the sclera and on the cornea, which extends spherical from the spherical sclera. Figure 13 further shows that the eye fixation portion 62 comprises one suction opening 621, which has a partial ring shape and which is connectable via a pressure line to a negative pressure. The suction opening 621 is according to this embodiment configured to be arranged on the cornea of the eye 91. In a further embodiment, the suction opening 621 might extends towards the sclera. In a further embodiment, the eye fixation portion 62, in particular may comprise one or a plurality of suction openings 621.

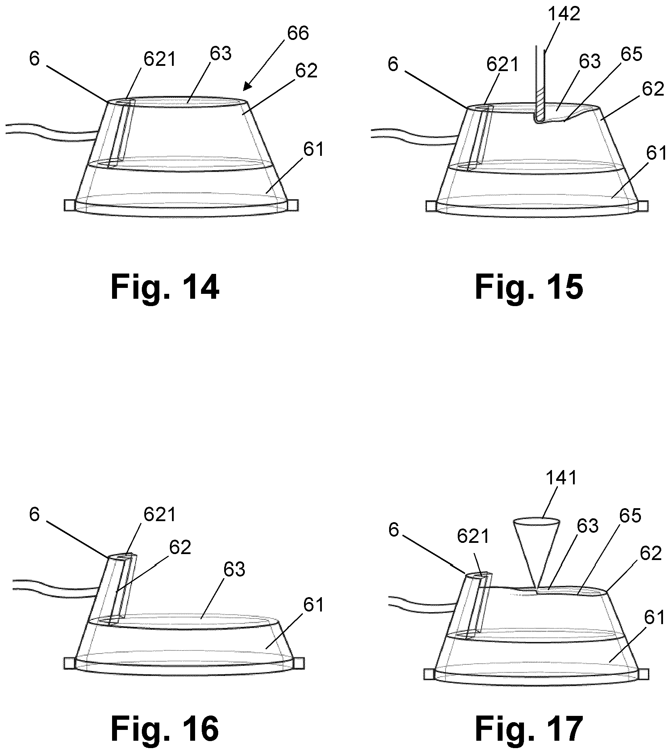

**Figure 14** and **Figure 15** shows an exemplary embodiment for manufacturing the patient-specific portion 65 of the ophthalmological patient interface 6 according to a seventh embodiment of the present disclosure. Figure 14 shows a blank unmachined ophthalmological patient interface 6. This blank ophthalmological patient interface 6 may have geometrical dimensions, which are limited by a predefined enveloping geometry 66 surrounding the ophthalmological patient interface 6. In other words, the predefined enveloping geometry 66 determines the maximal extensions of the blank unmachined ophthalmological patient interface 6. Figure 15 shows the manufacturing process of the patient-specific portion 65. According to this embodiment, the patient-specific portion 65 is manufactured by removing material using a removing device 142, for example a grinding machine or a milling machine. The patient-specific portion 65 is milled out of the blank eye fixation portion 62 of the ophthalmological patient interface 6. In another embodiment, the patient-specific portion 65 is milled out of the blank coupling portion 61 of the ophthalmological patient interface 6.

**Figure 16** and **Figure 17** shows an exemplary embodiment for manufacturing the patient-specific portion 65 of the ophthalmological patient interface 6 according to an eigth embodiment of the present disclosure. Figure 16 shows a blank unmachined ophthalmological patient interface 6. Figure 16 shows that the blank eye fixation portion 62 only comprises a portion for a suction opening 621, the rest of the eye fixation portion 62 is not jet formed. Figure 17 shows the manufacturing process of the patient-specific portion 65. According to this embodiment, the patient-specific portion 65 is manufactured by adding material using an additive manufacturing device 141, for example a 3D printer. A print head of the additive manufacturing device 141 is for example arranged on a six-axis jointed-arm robot. The patient-specific portion 65 is additively manufactured on the blank eye fixation portion 62 of the ophthalmological patient interface 6. In another embodiment, the patient-specific portion 65 is additively manufactured on a blank coupling portion 61 of the ophthalmological patient interface 6. In a further embodiment, also the suction opening 621 is additively formed by the additive manufacturing device 141. In a further embodiment, the entire ophthalmological patient interface 6 is formed additively by the additive manufacturing device 141.

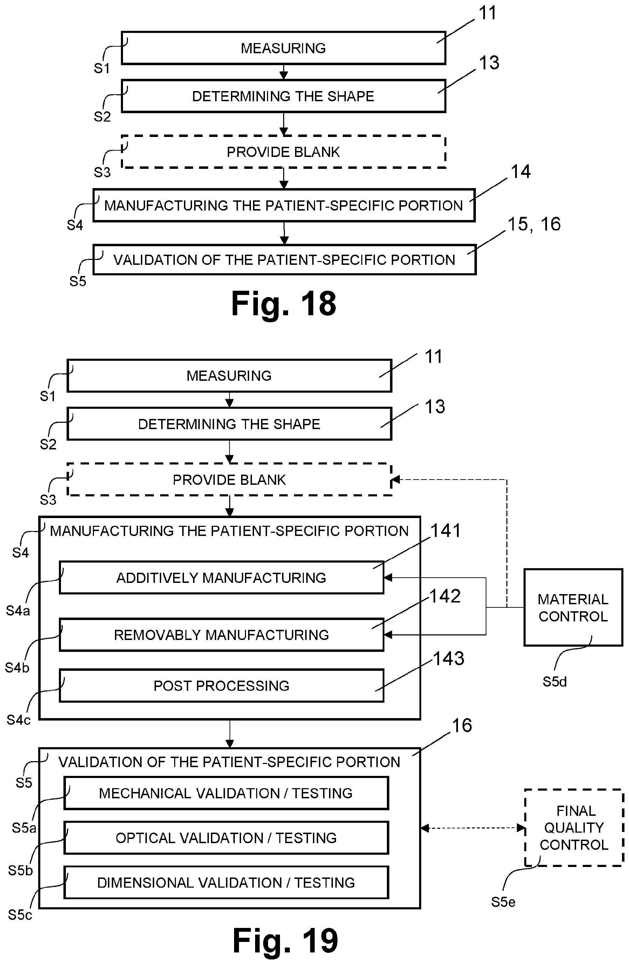

**Figure 18** and **Figure 19** shows a block diagram of a method of manufacturing an ophthalmological patient interface 6. The block diagram comprise different steps S1 to S5, which are performed for example by different devices.

In step S1, a measuring device 11 measures the surface of the eye 91 of the patient 9 and / or the eye surrounding surface 12 of the patient 9. The measuring device 11 may use different technologies to measure the surface of the eye 91 of the patient 9 and / or the eye surrounding surface 12 of the patient 9.

In step S2, a computing device 13 determines the shape of the surface of the eye 91 of the patient 9 and / or the shape of the eye surrounding surface 12 of the patient 9. The measured data of step S1 is, for example, transmitted from the measuring device 11 to the computing device 13, which determines for example a virtual model of the shape of the surface of the eye 91 and / or the shape of the eye surrounding surface 12 of the patient 9 using the received data. The measuring device 11 and the computing device 13 are for example a single device.

In the optional step S3, a blank coupling portion 61 and / or a blank eye fixation portion 62 is provided. For example, an ophthalmological patient interface 6 as shown in the Figures 14 or 16 is provided, which comprises a blank unmachined eye fixation portion 62. The provided blank may comprise a patient identifier, for example an rfid tag or a QR-code.

In step S4, a manufacturing device 14 manufactures the patient-specific portion 65 of the eye fixation portion 62 and / or the coupling portion 61, based on the determined shape of the surface of the eye 91 of the patient 9 or the shape of the eye surrounding surface 12 of the eye 9. For example, a software determines automatically or manually the desired shape of the patient-specific portion 65 using the provided data of step S2. The software creates, for example, a 3D model of the patient-specific portion 65 and / or the entire ophthalmological patient interface 6, wherein the dimensions of the patient specific portion 65 is for example within the predefined enveloping geometry.

In step S5, a certification software 15 and / or a quality validation device 16 validates at least one quality parameter of the manufactured patient-specific portion 65 of the individually shaped ophthalmological patient interface 6. The certification software 15 compares, for example, the outer contour of the manufactured patient-specific portion 65 with a virtual model of the desired patient-specific portion 65. In case the deviations between the virtual model and the manufactured patient-specific portion 65 reach or surpass a predefined threshold value, the manufactured patient-specific portion 65 may not pass the validation step. In another embodiment, the certification software 15 may monitor the measuring device 14, the computing device 13 and / or the manufacturing device 14. The quality validation device 16 is, for example, a separate device, which is configured for a mechanical, optical and / or dimensional validation of the finished patient-specific portion 65 of the ophthalmological patient interface 6.

Figure 19 deviates from Figure 18 with respect to step S4 and step S5. The manufacturing step S4 and the validation step S5 is presented in detail. The manufacturing step S4 comprises at least one of: an additively manufacturing step S4a or a removably manufacturing step S4b.

In the step S4a, an additive manufacturing device 141 manufactures additively the patient-specific portion 65 of the ophthalmological patient interface 6. In other words, the additive manufacturing device 141, for example a 3D printer, additively manufactures the patient-specific portion 65 of the eye fixation portion 62 and/or the coupling portion 61.

In the step S4b, a removing device 142 manufactures removably the patient-specific portion 65 of the ophthalmological patient interface 6. In other words, the removing device 142, for example a grinding or milling machine, forms the patient-specific portion 65 of the ophthalmological patient interface 6 by removing material. The steps S4a and S4b are for example performed in combination, preferably iteratively. In a further embodiment, one device comprises the additive manufacturing device 141 and the removing device 142.

In the step S4c, a post processing device 143 processes the additively and/or removably manufactured patient specific portion 65. The post processing step S4c is for example a finishing step, which may comprise heat treatment, hardening, polishing, liquid based surface treatment etc. The post processing step S4c may further comprise to add the patient identifier on the ophthalmological patient interface 6, for example, a QR-code is added via a laser on the ophthalmological patient interface 6. In another embodiment, the patient identifier may be additively or removably added in the steps S4a and / or S4b.

Figure 19 further shows the validation step S5 in detail. The step S5 comprises according to this embodiment a mechanical validation step S5a, an optical validation step S5b, a dimensional validation step S5c a material control step S5d and a final quality control step S5e.

In the step S5a, the quality validation device 16, performs a mechanical validation / testing of the patient-specific portion 65 and / or of the entire ophthalmological patient interface 6, for example mechanical load testing.

In the step S5b, the quality validation device 16, performs a optical validation / testing of the optical properties of the patient-specific portion 65 and / or of the entire ophthalmological patient interface 6, for example a transparency test of the contact body 626.

In the step S5c, the quality validation device 16, performs a dimensional validation / testing of the individual dimensional properties of the patient-specific portion 65 and / or of the entire ophthalmological patient interface 6, for example, the dimensions of the passage 63 or the dimensions of the eye contact surface is measured and validated.

In the step S5d, a material control is performed, for example via sample testing. The material control is for example performed on the provided blank in step S3, on the additively manufacturing step S4a and / or on the removable manufacturing step S4b. For example, the specific material used for additively manufacturing the patient specific portion is tested prior, for example prior usage.

In the step S5e, a final quality control is performed, for example, using an additional device or software.

In an embodiment, the optionally added patient identifier is scanned prior to the step of validation of the patient-specific portion 65 in order to identify and connect the validation results with the current validated patient-specific portion 65.

It should be noted that, in the description, the sequence of the steps has been presented in a specific order, one skilled in the art will understand, however, that the order of at least some of the steps could be altered or some steps could be skipped, without deviating from the scope of the disclosure.

### LIST OF REFERENCE SYMBOLS

- 100: ophthalmological laser treatment device
- 1: ophthalmological laser treatment device
- 2: base station
- 21: treatment laser source
- 22: scanner
- 23: optical module
- 3: control module
- 4: arm
- 41: first arm joint
- 42: second arm joint
- 43: third arm joint
- 5: laser applicator
- 51: focusing optics
- 6: patient interface
- 61: coupling portion
- 62: eye fixation portion
- 621: suction opening
- 622: contact surface
- 623: treatment surface
- 624: sealing
- 625: protrusion
- 626: contact body
- 627: extension portion
- 628: flat contact surface
- 629: form-fitted contact body
- 63: passage
- 65: patient-specific portion
- 66: enveloping geometry
- 7: optical imaging device
- 8: monitor
- 9: patient
- 91: eye of the patient
- 11: measuring device
- 12: eye surrounding surface
- 13: computing device
- 14: manufacturing device
- 141: additive manufacturing device
- 142: removing device
- 143: post processing device
- 15: certification software
- 16: quality validation device

- T: treatment laser beam
- v: optical axis of an eye
- p: main central optical axis of the laser applicator
- r: central axis of the coupling portion
- c: central axis of the eye fixation portion
- α: inclination angle

## Claims

1. Ophthalmological patient interface (6) for application to an eye (91) of a patient (9), the ophthalmological patient interface (6) comprising:
a. a coupling portion (61) configured to be arranged on a laser applicator (5) of an ophthalmological laser treatment device (1);
b. an eye fixation portion (62) configured to be arranged on the eye (91) of the patient (9);
c. a passage (63), extending through the coupling portion (61) and the eye fixation portion (62), wherein the passage (63) is configured to enable a treatment laser beam (T) from the laser applicator (5) to pass through the ophthalmological patient interface (6);
d. wherein at least one of: the coupling portion (61) or the eye fixation portion (62) comprises a patient-specific portion (65), shaped individually with respect to at least one of: a surface shape of the eye (91) of the patient (9) or a surface shape of a surrounding of the eye (91) of the patient (9).

2. The ophthalmological patient interface (6) according to claim 1, wherein the patient-specific portion (65) is individually shaped with respect to at least one of: the shape of the nose of the patient (9), the shape of the eye sockets of the patient (9), or the shape of deformities of the face of the patient (9) which surround the eye (91) of the patient (9).

3. The ophthalmological patient interface (6) according to one of the preceding claims, wherein the patient-specific portion (65) is further individually shaped with respect to a geometrical shape of the eye (91) of the patient (9), in particular with respect to at least one of: a diameter of the eye (91) of the patient (9), a curvature radius at a planned contact position of the eye (91) of the patient (9), an irregular curvature of the cornea of the eye (91) of the patient (9), an irregular curvature of the limbus of the eye (91) of the patient (9) or an irregular curvature of the sclera of the eye (91) of the patient (9).

4. The ophthalmological patient interface (6) according to one of the preceding claims, wherein the coupling portion (61) and the eye fixation portion (62) are two separate parts, which are configured to be coupled together to form the ophthalmological patient interface (6), wherein at least one of: the coupling portion (61) or the eye fixation portion (62), comprises the patient-specific portion (65).

5. The ophthalmological patient interface (6) according to one of the preceding claims, wherein the patient-specific portion (65) of the ophthalmological patient interface (6) is at least partially made of bio-compatible and / or sterilizable material.

6. The ophthalmological patient interface (6) according to one of the preceding claims, wherein the eye fixation portion (62) comprises an at least partially transparent contact body (626), which is arranged in the passage (63) at the eye facing end of the eye fixation portion (62), and which is configured to contact at least one of: the cornea or the sclera of the eye (91) of the patient (9), wherein the contact body (626) comprises the patient-specific portion (65).

7. The ophthalmological patient interface (6) according to one of the preceding claims, wherein at least one geometrical extension of the patient-specific portion (65) is limited to a predefined enveloping geometry (66) surrounding the ophthalmological patient interface (6).

8. The ophthalmological patient interface (6) according to one of the preceding claims, wherein the eye fixation portion (62) comprises a rotationally asymmetric contact surface (622) which is at least partially formed by the patient-specific portion (65), the rotationally asymmetric contact surface (622) being configured to contact at least one of: the sclera or the cornea of the eye (91) of the patient (9).

9. Method of manufacturing an ophthalmological patient interface (6) according to one of the preceding claims, the method comprising the following steps:
a. measuring (S1), by a measuring device (11), at least one of: the surface of the eye (91) of the patient (9) or the eye surrounding surface (12) of the patient (9);
b. determining (S2), by a computing device (13), at least one of: the shape of the surface of the eye (91) of the patient (9) or the shape of the eye surrounding surface (12) of the patient (9);
c. manufacturing (S4), by a manufacturing device (14), a patient-specific portion (65) of at least one of: the coupling portion (61) or the eye fixation portion (62), based on at least one of: the determined shape of the surface of the eye (91) of the patient (9) or the shape of the eye surrounding surface (12) of the eye (91) of the patient (9).

10. The method according to claim 9, wherein the step of manufacturing (S4a) comprises adding material, by an additive manufacturing device (141), for forming the patient-specific portion (65) of the ophthalmological patient interface (6).

11. The method according to claim 10, wherein the method further comprises the steps of:
a. providing (S3) at least one of: a blank coupling portion (61) or a blank eye fixation portion (62);
b. additively manufacturing (S4a), by the additive manufacturing device (141), the patient-specific portion (65) onto at least one of: the blank coupling portion (61) or the blank eye fixation portion (62), by adding material on at least one of the blank portion(s) (61, 62).

12. The method according to one of the claims 9 to 11, wherein the step of manufacturing (S4b) comprises removing material, by a removing device (142), for forming the patient-specific portion (65) of the ophthalmological patient interface (6).

13. The method according to claim 12, wherein the method further comprises the step of:
a. manufacturing (S4b), by the removing device (142), the patient-specific portion (65) out of at least one of: the blank coupling portion (61) or the blank eye fixation portion (62), by removing material from the blank portion(s).

14. The method according to one of the claims 9 to 13, wherein measuring (S1) is performed by at least one of: an optical coherence tomography device, a Scheimpflug tomography device, a placido topography, an optical biometer device, a 3D laser scanner device, a time of flight sensor or a structured illumination device.

15. The method according to one of the claims 9 to 14, further comprising the step of:
a. validating (S5), by at least one of: a certification software (15) or a quality validation device (16), the quality of the manufactured (S4) patient-specific portion (65) of the individually shaped ophthalmological patient interface (6).
